# EUROPÄISCHE PATENTANMELDUNG

(11) **EP 3 236 375 A2**
(43) Veröffentlichungstag der Anmeldung: **25.10.2017**
(21) Anmeldenummer: 17181767.9
(22) Anmeldetag: 18.07.2017
(51) Int. Cl.: G06F 19/00

(54) **VERFAHREN UND SYSTEM ZUM AUSGEBEN EINER MEDIZINISCHEN INFORMATION**

(30) Priorität: 24.08.2016 DE 102016215922
(71) Anmelder: Siemens Healthcare GmbH, 91052 Erlangen (DE)
(72) Erfinder: Allmendinger, Thomas, 91301 Forchheim (DE); Lerch, Daniel, 91365 Weilersbach (DE); Thierfelder, Carsten, 91361 Pinzberg (DE)

(57) **Zusammenfassung**

Die Erfindung betrifft ein Verfahren zum Ausgeben einer medizinischen Information, wobei die medizinische Information eine Eignung eines Patienten für eine Untersuchung mittels einer medizinischen Bildgebungsvorrichtung betrifft und/oder wobei die medizinische Information eine patientenspezifische Konfiguration einer medizinischen Bildgebungsvorrichtung, insbesondere für eine Untersuchung eines Patienten, betrifft, wobei das Verfahren die folgenden Schritte umfasst:
- Erfassen einer Messdatenreihe, welche zumindest einen physiologischen Parameter des Patienten betrifft, mittels eines ersten Wearables, welches von dem Patienten getragen wird,
- Ermitteln der medizinischen Information basierend auf der Messdatenreihe,
- Ausgeben der medizinischen Information.

## Beschreibung

Die Erfindung betrifft ein Verfahren zum Ausgeben einer medizinischen Information. Die Erfindung betrifft ferner ein System, aufweisend ein erstes Wearable. Die Erfindung betrifft ferner eine Verwendung eines ersten Wearables zum Ausgeben einer medizinischen Information. Die Erfindung betrifft ferner eine Verwendung eines Systems, aufweisend ein erstes Wearable und ein zweites Wearable, zum Ausgeben einer medizinischen Information.

Die Durchführung einer Untersuchung des Herzens mittels einer medizinischen Bildgebungsvorrichtung ist in vielen Fällen immer vergleichsweise komplex und ist im Ablauf wesentlich von der zur Verfügung stehenden Hardware der medizinischen Bildgebungsvorrichtung geprägt. Die an der medizinischen Bildgebungsvorrichtung optimal zu verwendenden Einstellungen hängen typischerweise stark von den individuellen Patienteneigenschaften, insbesondere der Herzrate und des Herzrhythmus, ab. Eine besondere Ausprägung dieses Problems ist, wenn auf Grund der physiologischen Parameter des Patienten, beispielsweise auf Grund einer sehr hohen Herzrate, einer arrhythmischen Herzrate oder ähnliches, die diagnostische Bildqualität der Untersuchung mit der zur Verfügung stehenden medizinischen Bildgebungsvorrichtung stark eingeschränkt ist. In einem solchen Fall kann, soweit es keine Kontraindikationen gibt, versucht werden, die Herzrate zu senken und insbesondere einen Sinusrhythmus herzustellen. Dazu können beispielsweise Betablocker, Natriumkanalblocker oder ähnliches verwendet werden. Bei der herkömmlichen Vorgehensweise erfolgt der überwiegende Teil der Prüfung der Herzrate, während sich der Patient auf einer Patientenlagerungsvorrichtung der medizinischen Bildgebungsvorrichtung befindet und beispielsweise mit einem EKG-Messsystem verbunden ist.

Mehrere Ansätze zur Unterstützung des Nutzers der medizinischen Bildgebungsvorrichtung bei der Auswahl und Anpassung von Untersuchungsparametern, die in Bezug auf eine Herzaktivität des Patienten optimiert sind, sind dem Fachmann beispielsweise aus US 8218719 B2 und US 8611987 B2 bekannt.

Da die medizinische Bildgebungsvorrichtung während der Prüfung der Herzrate des auf der Patientenlagerungsvorrichtung gelagerten Patienten typischerweise nicht für die Erzeugung von medizinischen Bildern genutzt wird, wird eine Herzbildgebung oft mit einem relativ langsamen Workflow in Verbindung gebracht und als den Durchsatz des Geräts beschränkend angesehen. Alternativ kann die Eignung des Patienten für eine Untersuchung des Herzens mit Hilfe eines EKG-Messsystems erfolgen, wobei sich der Patient nicht auf der Patientenlagerungsvorrichtung der medizinischen Bildgebungsvorrichtung befindet. In einem solchen Fall ist die Interpretation und Bewertung der von dem EKG-Messsystem ausgegebenen Ergebnisse im Hinblick auf die geplante Untersuchung typischerweise von einer entsprechend qualifizierten Bedienperson durchzuführen. Auf diese Weise kann zwar die Belegung der medizinischen Bildgebungsvorrichtung unterbunden werden, allerdings ist damit zusätzlicher Zeitaufwand für eine medizinische Fachkraft verbunden.

Die Erfindung hat die Aufgabe, eine Alternative zur herkömmlichen Prüfung einer Eignung eines Patienten für eine Untersuchung mittels einer medizinischen Bildgebungsvorrichtung bereitzustellen.

Jeder der Gegenstände der unabhängigen Ansprüche löst jeweils diese Aufgabe. In den abhängigen Ansprüchen sind weitere Ausführungsformen der Erfindung berücksichtigt.

Die Erfindung betrifft ein Verfahren zum Ausgeben einer medizinischen Information, wobei die medizinische Information eine Eignung eines Patienten für eine Untersuchung mittels einer medizinischen Bildgebungsvorrichtung betrifft und/oder wobei die medizinische Information eine patientenspezifische Konfiguration einer medizinischen Bildgebungsvorrichtung, insbesondere für eine Untersuchung eines Patienten, betrifft, wobei das Verfahren die folgenden Schritte umfasst:
- Erfassen einer Messdatenreihe, welche zumindest einen physiologischen Parameter des Patienten betrifft, mittels eines ersten Wearables, welches von dem Patienten getragen wird,
- Ermitteln der medizinischen Information basierend auf der Messdatenreihe,
- Ausgeben der medizinischen Information.

Insbesondere kann das erste Wearable eine erste Datenverarbeitungseinheit aufweisen. Insbesondere kann eine erste Software-Applikation von der ersten Datenverarbeitungseinheit ausgeführt werden. Insbesondere kann die medizinische Information mittels der ersten Software-Applikation ermittelt und/oder ausgegeben werden.

Insbesondere kann eine Referenzinformation bereitgestellt werden. Die Referenzinformation kann beispielsweise eine Identifikation der Untersuchung und/oder der medizinischen Bildgebungsvorrichtung betreffen. Die Referenzinformation kann beispielsweise eine untersuchungsspezifische Bedingung und/oder gerätespezifische Bedingung an den zumindest einen physiologischen Parameter des Patienten betreffen. Die medizinische Information kann beispielsweise basierend auf der Messdatenreihe und basierend auf der Referenzinformation ermittelt werden.

Insbesondere können Daten zwischen dem ersten Wearable und einem Datenverarbeitungssystem übertragen werden, wobei die Daten mindestens ein Element umfassen, welches aus der Gruppe gewählt ist, welche aus der Messdatenreihe, der medizinischen Information, der Referenzinformation und Kombinationen davon besteht.

Das Datenverarbeitungssystem kann beispielsweise eine zweite Datenverarbeitungseinheit aufweisen. Die zweite Software-Applikation kann beispielsweise von der zweiten Datenverarbeitungseinheit ausgeführt werden. Die medizinische Information kann beispielsweise mittels der zweiten Software-Applikation ermittelt und/oder ausgegeben werden.

Insbesondere kann das Datenverarbeitungssystem ein zweites Wearable sein und/oder aufweisen, welches von einer Bedienperson getragen wird. Insbesondere kann das zweite Wearable die zweite Datenverarbeitungseinheit aufweisen.

Insbesondere kann das erste Wearable eine erste Anzeigeeinheit aufweisen. Insbesondere kann das Datenverarbeitungssystem eine zweite Anzeigeeinheit aufweisen. Die medizinische Information kann beispielsweise mittels der ersten Anzeigeeinheit und/oder mittels der zweiten Anzeigeeinheit angezeigt werden.

Insbesondere können Daten zwischen dem ersten Wearable und der medizinischen Bildgebungsvorrichtung übertragen werden. Insbesondere können Daten zwischen dem Datenverarbeitungssystem, insbesondere dem zweiten Wearable, und der medizinischen Bildgebungsvorrichtung übertragen werden. Die Daten können beispielsweise mindestens ein Element umfassen, welches aus der Gruppe gewählt ist, welche aus der Messdatenreihe, der medizinischen Information, der Referenzinformation und Kombinationen davon besteht.

Insbesondere kann ein Untersuchungsparameter der medizinischen Bildgebungsvorrichtung basierend auf der medizinischen Information angepasst werden.

Insbesondere kann die medizinische Information eine erste Information umfassen, welche angibt, ob der Patient für die Untersuchung mittels der medizinischen Bildgebungsvorrichtung geeignet ist. Insbesondere kann die medizinische Information eine zweite Information umfassen, welche angibt, ob und/oder wie die Eignung des Patienten für die Untersuchung hergestellt und/oder verbessert werden kann. Insbesondere kann die medizinische Information eine dritte Information umfassen, welche eine Kenngröße des physiologischen Parameters des Patienten und/oder ein Maß für eine Abweichung einer Kenngröße des physiologischen Parameters des Patienten von einer Bezugsgröße betrifft. Insbesondere kann die medizinische Information eine vierte Information umfassen, welche angibt, ob und/oder wie die Konfiguration der medizinischen Bildgebungsvorrichtung für die Untersuchung des Patienten patientenspezifisch optimiert werden kann.

Insbesondere kann die Messdatenreihe eine Herzaktivität des Patienten betreffen.

Die Erfindung betrifft ferner ein System, aufweisend
- ein erstes Wearable mit einer Erfassungseinheit, welche zum Erfassen einer Messdatenreihe, welche zumindest einen physiologischen Parameter des Patienten betrifft, ausgebildet ist,
- eine Ermittlungseinheit, welche zum Ermitteln einer medizinischen Information basierend auf der Messdatenreihe ausgebildet ist, wobei die medizinische Information eine Eignung eines Patienten für eine Untersuchung mittels einer medizinischen Bildgebungsvorrichtung betrifft und/oder wobei die medizinische Information eine patientenspezifische Konfiguration einer medizinischen Bildgebungsvorrichtung, insbesondere für eine Untersuchung eines Patienten, betrifft,
- eine Ausgabeeinheit, welche zum Ausgeben der medizinischen Information ausgebildet ist.

Insbesondere kann das System ferner die folgenden Komponenten aufweisen:
- ein Datenverarbeitungssystem,
- eine Datenübertragungseinheit, welche zum Übertragen von Daten zwischen dem ersten Wearable und dem Datenverarbeitungssystem ausgebildet ist,
- wobei das Datenverarbeitungssystem die Ermittlungseinheit und/oder die Ausgabeeinheit aufweist.

Insbesondere kann das erste Wearable die Ermittlungseinheit und/oder die Ausgabeeinheit aufweisen. Insbesondere kann das Datenverarbeitungssystem ein zweites Wearable aufweisen, welches die Ermittlungseinheit und/oder die Ausgabeeinheit aufweist.

Insbesondere kann das System ferner die folgenden Komponenten aufweisen:
- die medizinische Bildgebungsvorrichtung,
- eine Datenübertragungseinheit,
- eine Untersuchungsparameter-Anpassungseinheit, welche zum Anpassen zumindest eines Untersuchungsparameters der medizinischen Bildgebungsvorrichtung basierend auf der medizinischen Information ausgebildet ist.

Insbesondere kann die Datenübertragungseinheit zum Übertragen von Daten zwischen dem ersten Wearable und der medizinischen Bildgebungsvorrichtung ausgebildet sein. Insbesondere kann die Datenübertragungseinheit zum Übertragen von Daten zwischen dem Datenverarbeitungssystem, insbesondere dem zweiten Wearable, und der medizinischen Bildgebungsvorrichtung ausgebildet sein.

Eine Ausführungsform der Erfindung betrifft ein System gemäß einem der Aspekte, die in dieser Beschreibung und/oder in den Ansprüchen offenbart sind, welches zum Ausführen eines Verfahrens nach einem der Aspekte, die in dieser Beschreibung und/oder in den Ansprüchen offenbart sind, ausgebildet ist.

Die Erfindung betrifft ferner eine Verwendung eines ersten Wearables zum Ausgeben einer medizinischen Information, wobei die medizinische Information eine Eignung eines Patienten für eine Untersuchung mittels einer medizinischen Bildgebungsvorrichtung betrifft und/oder wobei die medizinische Information eine patientenspezifische Konfiguration einer medizinischen Bildgebungsvorrichtung, insbesondere für eine Untersuchung eines Patienten, betrifft,
- wobei eine Messdatenreihe, welche zumindest einen physiologischen Parameter des Patienten betrifft, mittels des ersten Wearables, welches von dem Patienten getragen wird, erfasst wird,
- wobei die medizinische Information basierend auf der Messdatenreihe ermittelt wird,
- wobei die medizinische Information ausgegeben wird.

Die Erfindung betrifft ferner eine Verwendung eines Systems, aufweisend ein erstes Wearable und ein zweites Wearable, zum Ausgeben einer medizinischen Information, wobei die medizinische Information eine Eignung eines Patienten für eine Untersuchung mittels einer medizinischen Bildgebungsvorrichtung betrifft und/oder wobei die medizinische Information eine patientenspezifische Konfiguration einer medizinischen Bildgebungsvorrichtung, insbesondere für eine Untersuchung eines Patienten, betrifft,
- wobei eine Messdatenreihe, welche zumindest einen physiologischen Parameter des Patienten betrifft, mittels des ersten Wearables, welches von dem Patienten getragen wird, erfasst wird,
- wobei die medizinische Information basierend auf der Messdatenreihe ermittelt wird,
- wobei die medizinische Information mittels des zweiten Wearables ausgegeben wird.

Insbesondere kann die medizinische Information mittels des ersten Wearables und/oder mittels des zweiten Wearables basierend auf der Messdatenreihe ermittelt werden. Insbesondere kann die medizinische Information mittels des ersten Wearables und/oder mittels des zweiten Wearables ausgegeben werden.

Die Erfinder haben erkannt, dass es vorteilhaft ist, eine kontinuierliche Beobachtung des Patienten hinsichtlich seiner Eignung für eine Untersuchung mittels einer medizinischen Bildgebungsvorrichtung über einen längeren Zeitraum zu ermöglichen, und dass es insbesondere nicht sinnvoll ist, eine medizinische Bildgebungsvorrichtung mit einem Patienten zu belegen, wenn bei der Prüfung der Eignung des Patienten festgestellt werden kann, dass der Patient nicht für die Untersuchung mittels der medizinischen Bildgebungsvorrichtung geeignet ist und/oder Maßnahmen durchzuführen sind, um die Eignung des Patienten für die Untersuchung herzustellen und/oder zu verbessern.

Die Erfindung ermöglicht die Verlagerung der Prüfung der Eignung des Patienten weg von der medizinischen Bildgebungsvorrichtung hin zu einer mobilen Lösung, die insbesondere kostengünstig und unabhängig von der medizinischen Bildgebungsvorrichtung realisierbar ist. Damit ist eine kontinuierliche Beobachtung des Patienten hinsichtlich seiner Eignung für eine Untersuchung mittels einer medizinischen Bildgebungsvorrichtung über einen längeren Zeitraum realisierbar, ohne das während dessen die medizinische Bildgebungsvorrichtung durch den Patienten, dessen Eignung überprüft wird, belegt wird. Gemäß einem Aspekt der Erfindung wird die Messdatenreihe erfasst, während sich der Patient in einer von der medizinischen Bildgebungsvorrichtung entfernten Position befindet und/oder während die medizinische Bildgebungsvorrichtung, insbesondere die Patientenlagerungsvorrichtung der medizinischen Bildgebungsvorrichtung, nicht von dem Patienten belegt wird.

Bei der erfindungsgemäßen Lösung wird die medizinische Bildgebungsvorrichtung nicht unnötig durch Vorbereitungsmaßnahmen blockiert, so dass ein erhöhter Patientendurchsatz mit der medizinischen Bildgebungsvorrichtung realisierbar ist. Insbesondere ermöglicht die erfindungsgemäße Lösung eine kontinuierliche Beobachtung der physiologischen Parameter des Patienten über einen längeren Zeitraum, ohne die medizinische Bildgebungsvorrichtung zu blockieren. Damit können bestimmte Pathologien, welche beispielsweise die Herzaktivität betreffen und/oder lediglich sporadisch oder in (psychischen) Belastungssituationen auftreten, besser erkannt werden. Dagegen ist bei einer herkömmlichen Prüfung einer Eignung eines Patienten für eine Untersuchung mittels einer medizinischen Bildgebungsvorrichtung die EKG-Messung auf ein relativ kurzes Zeitintervall beschränkt, um die Untersuchung nicht unnötig zu verzögern.

Unter einem Wearable kann insbesondere ein in die Kleidung eines Nutzers integriertes und/oder am Körper eines Nutzers tragbares Computersystem verstanden werden. Das Wearable kann insbesondere dazu ausgebildet sein, Daten, die den Nutzer und/oder dessen Umwelt betreffen, zu erfassen und/oder zu verarbeiten. Insbesondere kann das Wearable derart in die Kleidung des Nutzers integriert und/der derart am Körper des Nutzers tragbar sein, dass der Nutzer beide Hände frei hat und/oder dass das Sichtfeld des Nutzers nicht durch das Wearable eingeschränkt ist. Unter einem Nutzer kann beispielsweise ein Patient und/oder eine Bedienperson, insbesondere Arzt oder MTA, verstanden werden. Das Wearable kann beispielsweise eine Smartwatch sein und/oder eine Smartwatch aufweisen. Das Wearable kann beispielsweise ein Fitness-Tracker sein und/oder einen Fitness-Tracker aufweisen. Dem Fachmann sind Smartwatches bekannt, mit denen eine Messdatenreihe, welche zumindest einen physiologischen Parameter des Patienten betrifft, erfasst werden kann. Beispiele für solche Smartwatches sind Apple Watch, Sony SmartWatch 3 und Samsung Galaxy Gear.

Das Wearable kann beispielsweise in ein Kleidungsstück, insbesondere ein Hemd, integriert sein und/oder eine Komponente aufweisen, die in ein Kleidungsstück integriert ist. Das Wearable kann beispielsweise ein Armband aufweisen, wobei das Wearable mittels des Armbands an einem Arm des Nutzers getragen werden kann. Dabei kann das Wearable beispielsweise in Form einer Armbanduhr an einem Handgelenk des Nutzers oder in Form eines Fitnessarmbands an einem Arm des Nutzers getragen werden.

Das Wearable kann beispielsweise eine Baugruppe aufweisen, welche einen Bereich des Armbands bildet und/oder welche an dem Armband angeordnet ist. Die Baugruppe kann beispielsweise die Datenverarbeitungseinheit, eine Anzeigeeinheit, ein Datenübertragungsmodul zur insbesondere kabellosen Datenübertragung und eine Energieversorgungseinheit zur Versorgung der Komponenten des Wearables mit elektrischer Energie aufweisen. Das Wearable kann beispielsweise eine Datenverarbeitungseinheit aufweisen. Auf der Datenverarbeitungseinheit kann beispielsweise eine Software-Applikation installiert werden. Die Software-Applikation kann von der Datenverarbeitungseinheit ausgeführt werden.

Die erste Datenverarbeitungseinheit ist nach einem der Aspekte einer Datenverarbeitungseinheit, die in dieser Beschreibung und/oder in den Ansprüchen offenbart sind, ausgebildet. Die zweite Datenverarbeitungseinheit ist nach einem der Aspekte einer Datenverarbeitungseinheit, die in dieser Beschreibung und/oder in den Ansprüchen offenbart sind, ausgebildet. Die erste Datenverarbeitungseinheit und die zweite Datenverarbeitungseinheit können unterschiedlich ausgebildet sein.

Insbesondere kann eine Schnittstelle zur Übertragung von Informationen zwischen der ersten Software-Applikation und der zweiten Software-Applikation bereitgestellt werden. Insbesondere kann eine solche Schnittstelle auf dem Client-Server-Modell basieren. In einem Speicherbereich der Schnittstelle kann beispielsweise von der ersten Software-Applikation und/oder von der zweiten Software-Applikation eine Datei mit den zu übertragenden Informationen hinterlegt werden und von der ersten Software-Applikation und/oder von der zweiten Software-Applikation gelesen werden. Das Format der Datei kann beispielsweise derart universell gewählt werden, dass auf diese Weise Informationen auch zwischen einer ersten Software-Applikation und einer zweiten Software-Applikation, die von unterschiedlichen Herstellern bereitgestellt wurden, übertragen werden können. Insbesondere kann das erste Wearable und/oder das zweite Wearable mittels der ersten Software-Applikation und/oder der zweiten Software-Applikation konfigurierbar sein.

Das erste Wearable ist nach einem der Aspekte eines Wearables, die in dieser Beschreibung und/oder in den Ansprüchen offenbart sind, ausgebildet. Das zweite Wearable ist nach einem der Aspekte eines Wearables, die in dieser Beschreibung und/oder in den Ansprüchen offenbart sind, ausgebildet. Das erste Wearable und das zweite Wearable können unterschiedlich ausgebildet sein. Das zweite Wearable kann beispielsweise von einem Nutzer getragen werden. Unter der Messdatenreihe kann beispielsweise ein Input der ersten Software-Applikation und/oder ein Input der zweiten Software-Applikation verstanden werden, wobei die medizinische Information basierend auf dem Input ermittelt wird.

Das Wearable kann beispielsweise eine Sensoreinheit aufweisen, welche zum Messen des zumindest einen physiologischen Parameters des Patienten ausgebildet ist. Insbesondere kann die Sensoreinheit einen Kontakt mit einem Bereich der Oberfläche des Patienten bilden. Die Sensoreinheit kann insbesondere dazu ausgebildet sein, in dem Bereich der Oberfläche des Patienten ein elektrisches Signal, ein optisches Signal, eine akustisches Signal, eine Farbe, eine Bewegung, eine Temperatur, eine Temperaturänderung oder ähnliches oder Kombinationen davon zu erfassen. Die Sensoreinheit kann beispielsweise in ein Kleidungsstück, insbesondere ein Hemd, integriert sein und/oder eine Komponente aufweisen, die in ein Kleidungsstück integriert ist.

Die Untersuchung mittels der medizinischen Bildgebungsvorrichtung kann beispielsweise eine Herzbildgebung sein und/oder eine Herzbildgebung aufweisen. Die Messdatenreihe kann beispielsweise erfasst werden, indem die Herzaktivität mittels einer Sensoreinheit gemessen wird, welche zum Messen der Herzaktivität des Patienten ausgebildet ist. Messdatenreihe, welche zumindest einen physiologischen Parameter betrifft, kann insbesondere eine Messdatenreihe sein, welche eine Herzaktivität des Patienten betrifft. Bei dem zumindest einen physiologischen Parameter kann es sich insbesondere um einen oder mehrere Vitalparameter handeln. Bei dem zumindest einen physiologischen Parameter kann es sich insbesondere um einen oder mehrere Parameter handeln, welche die Herzaktivität des Patienten betreffen.

Beispiele für einen physiologischen Parameter insbesondere im Zusammenhang mit einer Herzaktivität sind eine Herzschlagfrequenz, eine Regelmäßigkeit des Herzschlags, ein Rhythmus des Herzschlags, eine Pulshärte, eine Pulsamplitude, eine Pulsanstiegssteilheit, ein Blutdruck, eine Blutdruckanstiegsgeschwindigkeit, ein Füllungsvolumen oder ähnliches oder Kombinationen davon. Weitere Beispiele für einen physiologischen Parameter sind eine Hautleitfähigkeit, eine Gehirnaktivität, eine Muskelaktivität, eine periphere Durchblutung, eine Atemfrequenz, eine Körpertemperatur, eine Sauerstoffsättigung, ein Blutzuckerwert, eine Bewegungsintensität, eine physische Hyperaktivität (Unruhe) oder ähnliches oder Kombinationen davon. Insbesondere kann mittels eines Messsystems ein Messdatensatz erfasst werden, welcher zumindest einen weiteren physiologischen Parameter des Patienten betrifft, welcher nicht mittels des ersten Wearables erfasst wird. Die medizinische Information kann beispielsweise basierend auf der Messdatenreihe und basierend auf dem Messdatensatz ermittelt werden.

Die medizinische Information kann beispielsweise ausgegeben werden, indem die medizinische Information mittels einer Anzeigeeinheit angezeigt wird und/oder indem die medizinische Information in einer Speichereinheit hinterlegt wird und/oder indem die medizinische Information per Datenübertragung, die insbesondere kabellos sein kann, übertragen wird.

Die Software-Applikation kann beispielsweise dazu ausgebildet sein, basierend auf der Messdatenreihe und/oder der Referenzinformation und/oder weiterer physiologischer Parameter und/oder einem Patientenparametersatz eine erste Information zu ermitteln, welche angibt, ob der Patient für die Untersuchung mittels der medizinischen Bildgebungsvorrichtung geeignet ist oder nicht. Der Patientenparametersatz kann beispielsweise Parameter aufweisen, welche das Geschlecht des Patienten, das Gewicht des Patienten, die Größe des Patienten und/oder das Alter des Patienten betreffen. Der Patientenparametersatz kann beispielsweise mit Hilfe einer elektronischen Gesundheitsakte ("EMR- electronic medical record", "EHR- electronic health record") bereitgestellt werden.

Die erste Information kann beispielsweise eine binäre Information sein, insbesondere eine Ja-Nein-Antwort. Beispielsweise kann die erste Information angeben, ob der Patient für eine Ca-Scoring-Untersuchung geeignet ist. In einem solchen Fall kann die Referenzinformation beispielsweise Bedingungen an den zumindest einen physiologischen Parameter des Patienten betreffen, die spezifisch für eine Ca-Scoring-Untersuchung sind. Beispielsweise kann die erste Information angeben, ob der Patient für eine craniale Computertomographie (CCT) geeignet ist. In einem solchen Fall kann die Referenzinformation beispielsweise Bedingungen an den zumindest einen physiologischen Parameter des Patienten betreffen, die spezifisch für eine craniale Computertomographie sind. Die Referenzinformation kann beispielsweise mittels der ersten Software-Applikation und/oder mittels der zweiten Software-Applikation und/oder mittels einer Steuerungsvorrichtung der medizinischen Bildgebungsvorrichtung erzeugt werden.

Die Software-Applikation kann beispielsweise dazu ausgebildet sein, basierend auf der Messdatenreihe und/oder der Referenzinformation und/oder weiterer physiologischer Parameter und/oder einem Patientenparametersatz eine zweite Information zu ermitteln, welche angibt, ob und/oder wie die Eignung des Patienten für die Untersuchung hergestellt und/oder verbessert werden kann. Die zweite Information kann beispielsweise angeben ob und/oder in welchem Maße und/oder wie eine Reduktion der Herzrate mit Hilfe von Betablockern und/oder die Herstellung eines regelmäßigen Rhythmus mit Hilfe von Antiarrhythmika realisierbar ist, insbesondere um eine Eignung des Patienten für die Untersuchung herzustellen.

Die Software-Applikation kann beispielsweise dazu ausgebildet sein, basierend auf der Messdatenreihe und/oder der Referenzinformation und/oder weiterer physiologischer Parameter und/oder einem Patientenparametersatz eine dritte Information zu ermitteln, welche eine Kenngröße des physiologischen Parameters des Patienten und/oder ein Maß für eine Abweichung einer Kenngröße des physiologischen Parameters des Patienten von einer Bezugsgröße betrifft. Insbesondere kann die Kenngröße des physiologischen Parameters mittels der Software-Applikation basierend auf der Messdatenreihe ermittelt werden. Insbesondere kann die medizinische Information die Kenngröße umfassen. Ein Beispiel für eine solche Kenngröße ist die Herzschlagfrequenz in Schlägen pro Minute, die insbesondere über ein Zeitintervall gemittelt sein kann. Insbesondere kann ein Warnsignal ausgegeben werden, falls die Abweichung einen Schwellwert überschreitet oder unterschreitet. Auf diese Weise ist eine Überwachung des Patienten realisierbar. Insbesondere kann es aufgrund der Gabe von z. B. Betablockern zu Nebenwirkungen kommen, welche ein Eingreifen des medizinischen Personals erfordern. Das Warnsignal kann beispielsweise mittels des Datenverarbeitungssystems, insbesondere mittels des zweiten Wearables, optisch und/oder akustisch ausgegeben werden.

Die Software-Applikation kann beispielsweise dazu ausgebildet sein, basierend auf der Messdatenreihe und/oder der Referenzinformation und/oder weiterer physiologischer Parameter und/oder einem Patientenparametersatz eine vierte Information zu ermitteln, welche angibt, ob und/oder wie die Konfiguration der medizinischen Bildgebungsvorrichtung für die Untersuchung des Patienten patientenspezifisch optimiert werden kann. Insbesondere kann basierend auf der vierten Information die medizinische Bildgebungsvorrichtung, insbesondere in Bezug auf den zumindest einen Untersuchungsparameter, patientenspezifisch konfiguriert werden.

Insbesondere kann die erste Software-Applikation in eine erste graphische Benutzeroberfläche eingebettet sein, welche beispielsweise auf der ersten Datenverarbeitungseinheit installiert ist und/oder welche beispielsweise mittels der ersten Anzeigeeinheit ausgegeben wird. Insbesondere kann die zweite Software-Applikation in eine zweite graphische Benutzeroberfläche eingebettet sein, welche beispielsweise auf der zweiten Datenverarbeitungseinheit installiert ist und/oder welche beispielsweise mittels der zweiten Anzeigeeinheit ausgegeben wird.

Die erste Software-Applikation ist nach einem der Aspekte einer Software-Applikation, die in dieser Beschreibung und/oder in den Ansprüchen offenbart sind, ausgebildet. Die zweite Software-Applikation ist nach einem der Aspekte einer Software-Applikation, die in dieser Beschreibung und/oder in den Ansprüchen offenbart sind, ausgebildet. Die erste Software-Applikation und die zweite Software-Applikation können unterschiedlich ausgebildet sein. Die untersuchungsspezifische Bedingung kann beispielsweise spezifisch für die Untersuchung des Patienten mittels der medizinischen Bildgebungsvorrichtung sein. Die gerätespezifische Bedingung kann beispielsweise spezifisch für die medizinische Bildgebungsvorrichtung sein. Die patientenspezifische Konfiguration der medizinischen Bildgebungsvorrichtung kann beispielsweise spezifisch für den Patienten sein.

Eine Datenverarbeitungseinheit kann beispielsweise eine oder mehrere Komponenten in Form von Hardware und/oder eine oder mehrere Komponenten in Form von Software aufweisen. Das Datenverarbeitungssystem kann beispielsweise eine oder mehrere Komponenten in Form von Hardware und/oder eine oder mehrere Komponenten in Form von Software aufweisen. Das Datenverarbeitungssystem kann beispielsweise zumindest teilweise von einem Cloud-Computing-System gebildet sein. Das Datenverarbeitungssystem kann beispielsweise ein Cloud-Computing-System, ein Computernetzwerk, ein Computer, ein Tabletcomputer, ein Smartphone oder ähnliches oder Kombinationen davon sein und/oder aufweisen. Die Hardware kann beispielsweise mit einer Software zusammenwirken und/oder mittels einer Software konfigurierbar sein. Die Software kann beispielsweise mittels der Hardware ausgeführt werden. Bei der Hardware kann es sich beispielsweise um ein Speichersystem, ein FPGA-System (Fieldprogrammable gate array), ein ASIC-System (Applicationspecific integrated circuit), ein Mikrocontroller-System, ein Prozessorsystem und Kombinationen davon handeln. Das Prozessorsystem kann beispielsweise einen Mikroprozessor und/oder mehrere zusammenwirkende Mikroprozessoren aufweisen.

Insbesondere kann eine Komponente eines Systems nach einem der Aspekte, die in dieser Beschreibung und/oder in den Ansprüchen offenbart sind, welche dazu ausgebildet ist, einen gegebenen Schritt eines Verfahrens nach einem der Aspekte, die in dieser Beschreibung und/oder in den Ansprüchen offenbart sind, auszuführen, in Form einer Hardware implementiert sein, welche zum Ausführen des gegebenen Schritts konfiguriert ist und/oder welche zum Ausführen einer computerlesbaren Anweisung derart konfiguriert ist, dass die Hardware mittels der computerlesbaren Anweisung zum Ausführen des gegebenen Schritts konfigurierbar ist. Insbesondere kann das System einen Speicherbereich, beispielsweise in Form eines computerlesbaren Mediums, aufweisen, in welchem computerlesbare Anweisungen, beispielsweise in Form eines Computerprogramms, gespeichert sind.

Ein Datentransfer zwischen Komponenten der Datenverarbeitungseinheit kann beispielsweise jeweils mittels einer geeigneten Datentransfer-Schnittstelle erfolgen. Die Datentransfer-Schnittstelle zum Datentransfer an und/oder von einer Komponente der Datenverarbeitungseinheit kann zumindest teilweise in Form von Software und/oder zumindest teilweise in Form von Hardware realisiert sein. Die Datentransfer-Schnittstelle kann beispielsweise zum Abspeichern von Daten in und/oder zum Laden von Daten aus einem Bereich des Speichersystems ausgebildet sein, wobei auf diesen Bereich des Speichersystems eine oder mehrere Komponenten der Datenverarbeitungseinheit zugreifen können.

Die medizinische Bildgebungsvorrichtung kann beispielsweise aus der Bildgebungsmodalitäten-Gruppe gewählt sein, welche aus einem Röntgengerät, einem C-Bogen-Röntgengerät, einem Computertomographiegerät (CT-Gerät), einem Molekularbildgebungsgerät (MI-Gerät), einem Einzelphotonen-Emissions-Computertomographiegerät (SPECT-Gerät), einem Positronen-Emissions-Tomographiegerät (PET-Gerät), einem Magnetresonanztomographiegerät (MRT-Gerät) und Kombinationen daraus (insbesondere PET-CT-Gerät, PET-MR-Gerät) besteht. Die medizinische Bildgebungsvorrichtung kann ferner eine Kombination einer Bildgebungsmodalität, die beispielsweise aus der Bildgebungsmodalitäten-Gruppe gewählt ist, und einer Bestrahlungsmodalität aufweisen. Dabei kann die Bestrahlungsmodalität beispielsweise eine Bestrahlungseinheit zur therapeutischen Bestrahlung aufweisen. Ohne Einschränkung des allgemeinen Erfindungsgedankens wird bei einigen der Ausführungsformen ein Computertomographiegerät beispielhaft für eine medizinische Bildgebungsvorrichtung genannt.

Gemäß einer Ausführungsform der Erfindung weist die medizinische Bildgebungsvorrichtung eine Akquisitionseinheit, welche zur Akquisition der Akquisitionsdaten ausgebildet ist, auf. Insbesondere kann die Akquisitionseinheit eine Strahlungsquelle und einen Strahlungsdetektor aufweisen. Eine Ausführungsform der Erfindung sieht vor, dass die Strahlungsquelle zur Emission und/oder zur Anregung einer Strahlung, insbesondere einer elektromagnetischen Strahlung, ausgebildet ist und/oder dass der Strahlungsdetektor zur Detektion der Strahlung, insbesondere der elektromagnetischen Strahlung, ausgebildet ist. Die Strahlung kann beispielsweise von der Strahlungsquelle zu einem abzubildenden Bereich gelangen und/oder nach einer Wechselwirkung mit dem abzubildenden Bereich zu dem Strahlungsdetektor gelangen. Bei der Wechselwirkung mit dem abzubildenden Bereich wird die Strahlung modifiziert und damit zum Träger von Informationen, die den abzubildenden Bereich betreffen. Bei der Wechselwirkung der Strahlung mit dem Detektor werden diese Informationen in Form von Akquisitionsdaten erfasst.

Insbesondere bei einem Computertomographiegerät und bei einem C-Bogen-Röntgengerät können die Akquisitionsdaten Projektionsdaten, die Akquisitionseinheit eine Projektionsdaten-Akquisitionseinheit, die Strahlungsquelle eine Röntgenquelle, der Strahlungsdetektor ein Röntgendetektor sein. Der Röntgendetektor kann insbesondere ein quantenzählender und/oder energieauflösender Röntgendetektor sein.

Insbesondere bei einem Magnetresonanztomographiegerät können die Akquisitionsdaten ein Magnetresonanzdatensatz, die Akquisitionseinheit eine Magnetresonanzdaten-Akquisitionseinheit, die Strahlungsquelle eine erste Hochfrequenzantenneneinheit, der Strahlungsdetektor die erste Hochfrequenzantenneneinheit und/oder eine zweite Hochfrequenzantenneneinheit sein.

Im Rahmen der Erfindung können Merkmale, welche in Bezug auf unterschiedliche Ausführungsformen der Erfindung und/oder unterschiedliche Anspruchskategorien (Verfahren, Vorrichtung, System usw.) beschrieben sind, zu weiteren Ausführungsformen der Erfindung kombiniert werden. Mit anderen Worten können die gegenständlichen Ansprüche auch mit den Merkmalen, die in Zusammenhang mit einem Verfahren beschrieben oder beansprucht sind, weitergebildet sein. Funktionale Merkmale eines Verfahrens können dabei durch entsprechend ausgebildete gegenständliche Komponenten ausgeführt werden. Neben den in dieser Anmeldung ausdrücklich beschriebenen Ausführungsformen der Erfindung sind vielfältige weitere Ausführungsformen der Erfindung denkbar, zu denen der Fachmann gelangen kann, ohne den Bereich der Erfindung zu verlassen, soweit er durch die Ansprüche vorgegeben ist.

Die Verwendung der unbestimmten Artikel "ein" bzw. "eine" schließt nicht aus, dass das betroffene Merkmal auch mehrfach vorhanden sein kann. Die Verwendung des Ausdrucks "aufweisen" schließt nicht aus, dass die mittels des Ausdrucks "aufweisen" verknüpften Begriffe identisch sein können. Beispielsweise weist die medizinische Bildgebungsvorrichtung die medizinische Bildgebungsvorrichtung auf. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander separiert sind.

Die Verwendung von Ordnungszahlwörtern (erste, zweite, dritte etc.) in der Bezeichnung von Merkmalen dient im Kontext der vorliegenden Anmeldung vor allem der besseren Unterscheidbarkeit der unter Verwendung von Ordnungszahlwörtern bezeichneten Merkmale. Das Nichtvorhandensein eines Merkmals, welches durch eine Kombination eines gegebenen Ordnungszahlworts und eines Begriffs bezeichnet wird, schließt nicht aus, dass ein Merkmal vorhanden sein kann, welches durch eine Kombination eines dem gegebenen Ordnungszahlwort nachfolgenden Ordnungszahlworts und des Begriffs bezeichnet wird.

Der Ausdruck "basierend auf" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal erzeugt (alternativ: ermittelt, bestimmt etc.) wird, nicht aus, dass das erste Merkmal basierend auf einem dritten Merkmal erzeugt (alternativ: ermittelt, bestimmt etc.) werden kann.

Im Folgenden werden ausgewählte Ausführungsformen unter Hinweis auf die beigefügten Figuren erläutert. Die Darstellung in den Figuren ist schematisch, stark vereinfacht und nicht zwingend maßstabsgetreu.

Es zeigen:
Fig. 1 ein Ablaufdiagramm für ein Verfahren zum Ausgeben einer medizinischen Information gemäß einer Ausführungsform der Erfindung,
Fig. 2 ein Ablaufdiagramm für ein Verfahren zum Ausgeben einer medizinischen Information gemäß einer weiteren Ausführungsform der Erfindung,
Fig. 3 ein System gemäß einer Ausführungsform der Erfindung,
Fig. 4 ein System gemäß einer weiteren Ausführungsform der Erfindung,
Fig. 5 ein System mit einem ersten Wearable gemäß einer weiteren Ausführungsform der Erfindung,
Fig. 6 ein System mit einem ersten Wearable und einem zweiten Wearable gemäß einer weiteren Ausführungsform der Erfindung,
Fig. 7 ein System mit einer Mehrzahl von ersten Wearables und einem zweiten Wearable gemäß einer weiteren Ausführungsform der Erfindung, und
Fig. 8 ein System mit einer medizinischen Bildgebungsvorrichtung gemäß einer weiteren Ausführungsform der Erfindung.

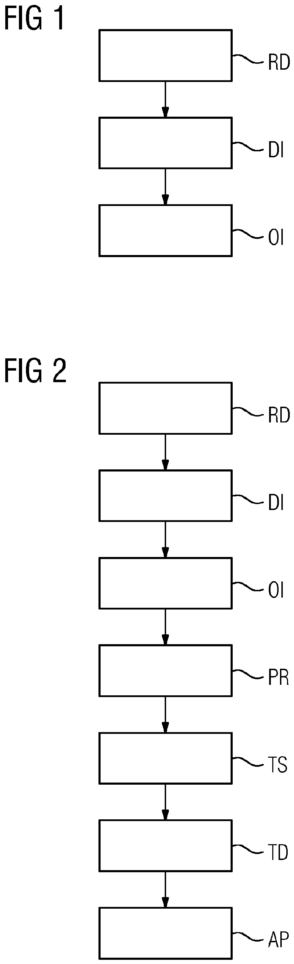

Fig. 1 zeigt ein Ablaufdiagramm für ein Verfahren zum Ausgeben einer medizinischen Information gemäß einer Ausführungsform der Erfindung, wobei das Verfahren die folgenden Schritte umfasst:
- Erfassen RD einer Messdatenreihe, welche zumindest einen physiologischen Parameter des Patienten 13 betrifft, mittels eines ersten Wearables W1, welches von dem Patienten 13 getragen wird,
- Ermitteln DI der medizinischen Information, welche eine Eignung eines Patienten 13 für eine Untersuchung mittels einer medizinischen Bildgebungsvorrichtung 2 und/oder eine patientenspezifische Konfiguration einer medizinischen Bildgebungsvorrichtung 2, insbesondere für eine Untersuchung eines Patienten 13, betrifft, basierend auf der Messdatenreihe,
- Ausgeben OI der medizinischen Information.

Fig. 2 zeigt ein Ablaufdiagramm für ein Verfahren zum Ausgeben einer medizinischen Information gemäß einer weiteren Ausführungsform der Erfindung, wobei das Verfahren ferner die folgenden Schritte umfasst:
- Bereitstellen PR einer Referenzinformation, welche eine Identifikation der Untersuchung und/oder der medizinischen Bildgebungsvorrichtung 2 betrifft und/oder welche eine untersuchungsspezifische und/oder gerätespezifische Bedingung an den zumindest einen physiologischen Parameter des Patienten 13 betrifft,
- Übertragen TS von Daten zwischen dem ersten Wearable W1 und einem Datenverarbeitungssystem,
- Übertragen TD von Daten zwischen dem ersten Wearable W1 und der medizinischen Bildgebungsvorrichtung 2 und/oder zwischen dem Datenverarbeitungssystem und der medizinischen Bildgebungsvorrichtung 2,
- Anpassen AP zumindest eines Untersuchungsparameters der medizinischen Bildgebungsvorrichtung 2 basierend auf der medizinischen Information. Dabei wird die medizinische Information basierend auf der Messdatenreihe und basierend auf der Referenzinformation ermittelt. Dabei umfassen die Daten mindestens ein Element, welches aus der Gruppe gewählt ist, welche aus der Messdatenreihe, der medizinischen Information, der Referenzinformation und Kombinationen davon besteht.

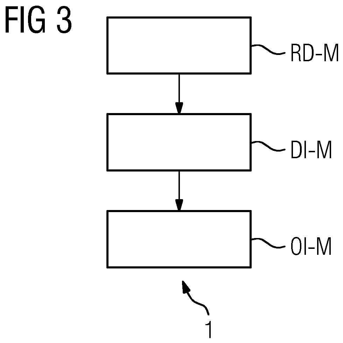

Fig. 3 zeigt ein System 1 gemäß einer Ausführungsform der Erfindung, aufweisend
- eine Erfassungseinheit RD-M,
- eine Ermittlungseinheit DI-M und
- eine Ausgabeeinheit OI-M.

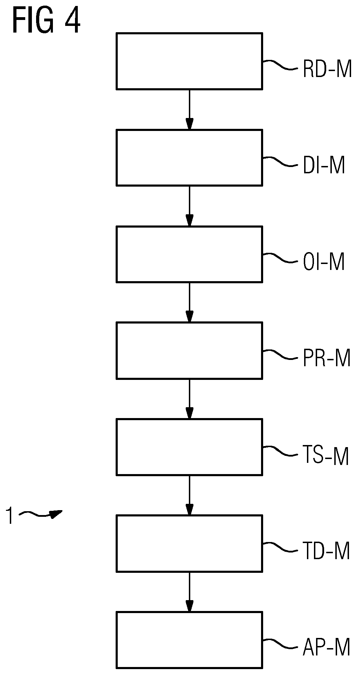

Fig. 4 zeigt ein System 1 gemäß einer weiteren Ausführungsform der Erfindung, ferner aufweisend
- eine Referenzinformation-Bereitstellungseinheit PR-M, welche zum Bereitstellen PR der Referenzinformation ausgebildet ist,
- eine Datenübertragungseinheit TS-M, welche zum Übertragen TS von Daten zwischen dem ersten Wearable W1 und dem Datenverarbeitungssystem ausgebildet ist,
- eine Datenübertragungseinheit TD-M, welche zum Übertragen TD von Daten zwischen dem ersten Wearable W1 und der medizinischen Bildgebungsvorrichtung 2 und/oder zwischen dem Datenverarbeitungssystem und der medizinischen Bildgebungsvorrichtung 2 ausgebildet ist, und
- eine Untersuchungsparameter-Anpassungseinheit AP-M, welche zum Anpassen AP zumindest eines Untersuchungsparameters der medizinischen Bildgebungsvorrichtung 2 basierend auf der medizinischen Information ausgebildet ist.

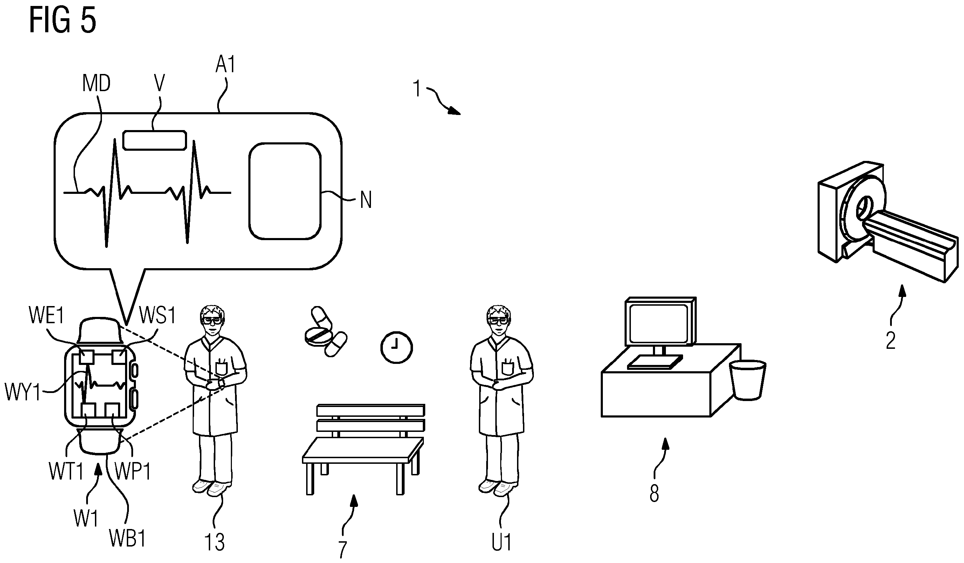

Fig. 5 zeigt ein System 1 mit einem ersten Wearable gemäß einer weiteren Ausführungsform der Erfindung. Die erste Software-Applikation A1 wird von der ersten Datenverarbeitungseinheit WP1 des ersten Wearables W1 ausgeführt. Die medizinische Information wird mittels der ersten Software-Applikation A1 basierend auf der Messdatenreihe MD ermittelt und ausgegeben. Das erste Wearable W1 wird in Form einer Smartwatch von dem Patienten 13 getragen. Das erste Wearable W1 weist die folgenden Komponenten auf: das erste Armband WB1, die erste Sensoreinheit WS1, die erste Datenverarbeitungseinheit WP1, die erste Anzeigeeinheit WY1, das zweite Datenübertragungsmodul WT1 zur insbesondere kabellosen Datenübertragung und die erste Energieversorgungseinheit WE1, beispielsweise in Form einer wiederaufladbaren elektrischen Batterie, zur Versorgung der Komponenten des ersten Wearables W1 mit elektrischer Energie.

Die medizinische Information N kann mittels der ersten Software-Applikation A1 ausgegeben werden, indem die medizinische Information N mittels der ersten Anzeigeeinheit WY1 des ersten Wearables W1 angezeigt wird. Optional kann die Messdatenreihe MD mittels der ersten Software-Applikation A1 ausgegeben werden, indem die Messdatenreihe MD mittels der ersten Anzeigeeinheit WY1 des ersten Wearables W1 angezeigt wird. Mittels der ersten Anzeigeeinheit WY1 kann die Messdatenreihe MD und/oder die medizinische Information N und/oder ein Wert V einer Kenngröße eines physiologischen Parameters angezeigt werden. Bei dem Wert V kann es sich insbesondere um die Herzschlagfrequenz des Patienten 13 handeln, die beispielsweise 63 Schläge pro Minute sein kann.

Der Patient 13 befindet sich in einem Patientenaufenthaltsbereich 7, beispielsweise einem Warteraum. Die Bedienperson U1, beispielsweise ein Arzt oder medizinisch-technischer Assistent (MTA), kann die medizinische Information N durch Beobachten der ersten Anzeigeeinheit zur Kenntnis nehmen. Die Bedienperson U1 kann die erste Anzeigeeinheit WY1 des ersten Wearables W1 beobachten, wenn sie sich in der Nähe des Patienten 13 aufhält. In der Regel ist es dafür erforderlich, dass die Bedienperson sich von dem Arbeitsbereich 8 entfernt. Der Arbeitsbereich 8 kann beispielsweise ein Konsolenraum, in dem sich eine Konsole zur Steuerung der medizinischen Bildgebungsvorrichtung 2 befindet, und/oder ein Untersuchungsraum, in dem sich die medizinische Bildgebungsvorrichtung 2 befindet, sein. Insbesondere falls Maßnahmen zur Herstellung und/oder Verbesserung der Eignung des Patienten 13 eingeleitet wurden, beispielsweise dem Patienten 13 Betablocker verabreicht wurden, kann die Bedienperson durch Beobachten der ersten Anzeigeeinheit in regelmäßigen Abständen den Verlauf der physiologischen Parameter beobachten und/oder die Eignung des Patienten 13 feststellen.

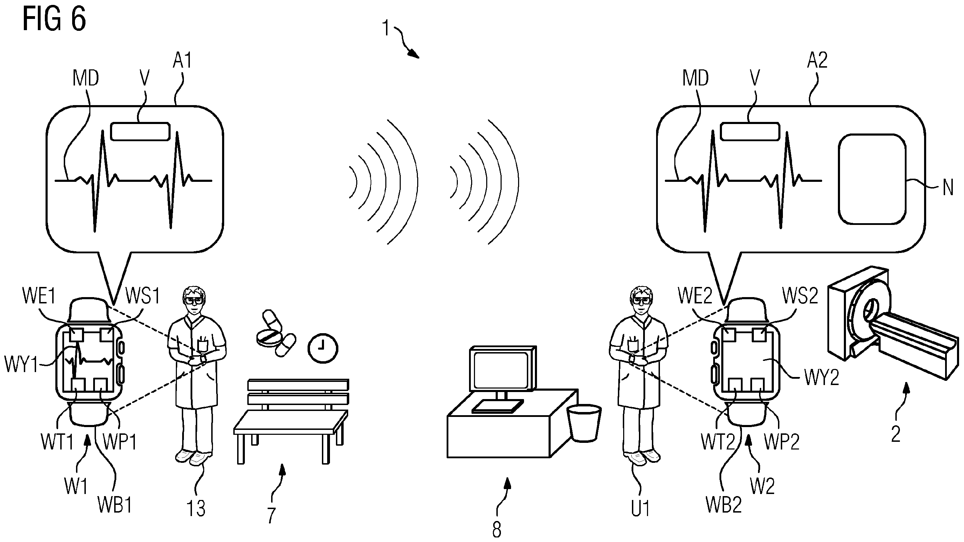

Fig. 6 zeigt ein System 1 mit einem ersten Wearable und einem zweiten Wearable gemäß einer weiteren Ausführungsform der Erfindung. Bei der in der Fig. 6 gezeigten Ausführungsform der Erfindung wird die medizinische Information N und die Messdatenreihe MD von dem ersten Wearable W1 an das zweite Wearable W2 übertragen. Das zweite Wearable W2 wird in Form einer Smartwatch von der Bedienperson U1 getragen. Das zweite Wearable W1 weist die folgenden Komponenten auf: das zweite Armband WB2, die zweite Sensoreinheit WS2, die zweite Datenverarbeitungseinheit WP2, die zweite Anzeigeeinheit WY2, das zweite Datenübertragungsmodul WT2 zur insbesondere kabellosen Datenübertragung und die zweite Energieversorgungseinheit WE2, beispielsweise in Form einer wiederaufladbaren elektrischen Batterie, zur Versorgung der Komponenten des zweiten Wearables W2 mit elektrischer Energie.

Von der zweiten Datenverarbeitungseinheit WP2 des zweiten Wearables W2 wird die zweite Software-Applikation A2 ausgeführt. Die medizinische Information N kann mittels der zweiten Software-Applikation A2 ausgegeben werden, indem die medizinische Information N mittels der zweiten Anzeigeeinheit WY2 des zweiten Wearables W2 angezeigt wird. Optional kann die Messdatenreihe MD mittels der zweiten Software-Applikation A2 ausgegeben werden, indem die Messdatenreihe MD mittels der zweiten Anzeigeeinheit WY2 des zweiten Wearables W2 angezeigt wird. Mittels der zweiten Anzeigeeinheit WY2 kann die Messdatenreihe MD und/oder die medizinische Information N und/oder ein Wert V einer Kenngröße eines physiologischen Parameters angezeigt werden. Auf diese Weise kann die medizinische Information N und/oder die Messdatenreihe MD von der Bedienperson beobachtet werden, während sie weitere Tätigkeiten, beispielsweise in dem Arbeitsbereich 8, ausführt und/oder während sie sich nicht in demselben Raum, wie der Patient, dessen Eignung überprüft wird, befindet.

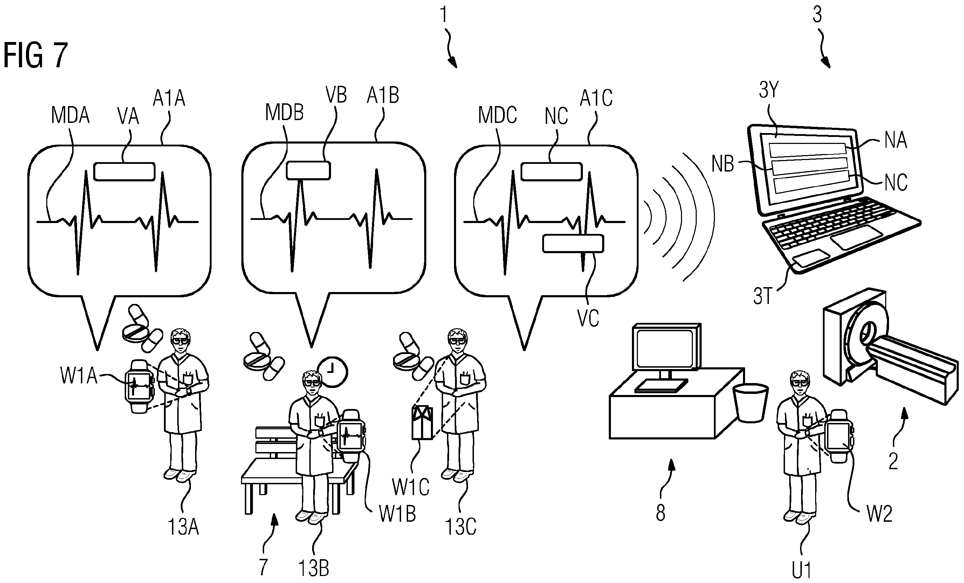

Fig. 7 zeigt ein System 1 mit einer Mehrzahl von ersten Wearables und einem zweiten Wearable gemäß einer weiteren Ausführungsform der Erfindung. Bei der in der Fig. 7 gezeigten Ausführungsform der Erfindung wird für jeden Patienten einer Mehrzahl von Patienten jeweils eine Messdatenreihe, welche zumindest einen physiologischen Parameter des jeweiligen Patienten betrifft, mittels eines ersten Wearables, welches von dem jeweiligen Patienten getragen wird, erfasst. Jeder Patient der Mehrzahl von Patienten trägt jeweils zumindest ein erstes Wearable, welches nach einem der Aspekte eines Wearables, die in dieser Beschreibung und/oder in den Ansprüchen offenbart sind, ausgebildet ist. Insbesondere können sich die Wearables W1A, W1B, W1C voneinander unterscheiden.

Der Patient 13A trägt das erste Wearable W1A in Form einer Smartwatch, welches eine Datenverarbeitungseinheit aufweist, von der die erste Software-Applikation A1A ausgeführt wird. Mittels des ersten Wearables W1A wird die Messdatenreihe MDA erfasst. Der Wert VA ist die Herzschlagfrequenz des Patienten 13A, beispielsweise 63 Schläge pro Minute. Der Patient 13B trägt das erste Wearable W1B in Form einer Smartwatch, welches eine Datenverarbeitungseinheit aufweist, von der die erste Software-Applikation A1B ausgeführt wird. Mittels des ersten Wearables W1B wird die Messdatenreihe MDB erfasst. Der Wert VB ist die Herzschlagfrequenz des Patienten 13B, beispielsweise 76 Schläge pro Minute. Der Patient 13C trägt das erste Wearable W1C, welches eine Datenverarbeitungseinheit aufweist, von der die erste Software-Applikation A1C ausgeführt wird. Mittels des ersten Wearables W1C wird die Messdatenreihe MDC erfasst. Der Wert VC ist die Herzschlagfrequenz des Patienten 13C, beispielsweise 92 Schläge pro Minute. Das Wearable W1C ist in ein Kleidungsstück, beispielsweise ein Hemd, welches der Patient 13C trägt, integriert.

Das Erfassen der Messdatenreihen MDA, MDB und MDC kann insbesondere parallel erfolgen. Für jeden Patienten der Mehrzahl von Patienten wird eine medizinische Information basierend auf der jeweiligen Messdatenreihe MDA, MDB, MDC ermittelt, wobei die medizinische Information eine Eignung des jeweiligen Patienten für eine Untersuchung mittels der medizinischen Bildgebungsvorrichtung 2 und/oder eine patientenspezifische Konfiguration der medizinischen Bildgebungsvorrichtung 2 für eine Untersuchung des jeweiligen Patienten betrifft. Für jeden Patient der Mehrzahl von Patienten wird die medizinische Information an das Datenverarbeitungssystem übertragen, welches den Computer 3 und das zweite Wearable W2 aufweist.

Der Computer 3 weist insbesondere den Bildschirm 3Y und das Datenübertragungsmodul 3T auf. Mittels des Bildschirms 3Y des Computers 3 können die medizinische Information NA, welche den Patienten 13A betrifft, die medizinische Information NB, welche den Patienten 13B betrifft, und die medizinische Information NC, welche den Patienten 13C betrifft, gleichzeitig angezeigt werden. Die Datenübertragungseinheit TS-M wird von dem Datenübertragungsmodul WT1 und zumindest einem der Datenübertragungsmodule WT2 und 3T gebildet. Die Datenübertragungseinheit TS-M kann beispielsweise auf kabelloser Datenübertragung und/oder einem Funknetz basieren und/oder zumindest eine Basisstation, mit welcher die Datenübertragungsmodule WT1, WT2 und 3T jeweils verbunden sein können, aufweisen.

Insbesondere kann mittels des Datenverarbeitungssystems eine medizinische Information, beispielsweise basierend auf einer Abweichung einer Kenngröße des physiologischen Parameters von einer Bezugsgröße, ausgewählt werden. Dabei kann es sich beispielsweise um die medizinische Information NC für einen Patienten 13C handeln, dessen Herzschlagfrequenz einen Schwellwert überschreitet. Die ausgewählte medizinische Information kann der Bedienperson mittels der zweiten Anzeigeeinheit WY2 des zweiten Wearables W2 angezeigt werden. Ferner kann basierend auf den medizinischen Informationen eine Reihenfolge der Patienten für die Untersuchung mittels der medizinischen Bildgebungsvorrichtung 2 ermittelt werden und/oder ein Patient für die Untersuchung mittels der medizinischen Bildgebungsvorrichtung 2 ausgewählt werden.

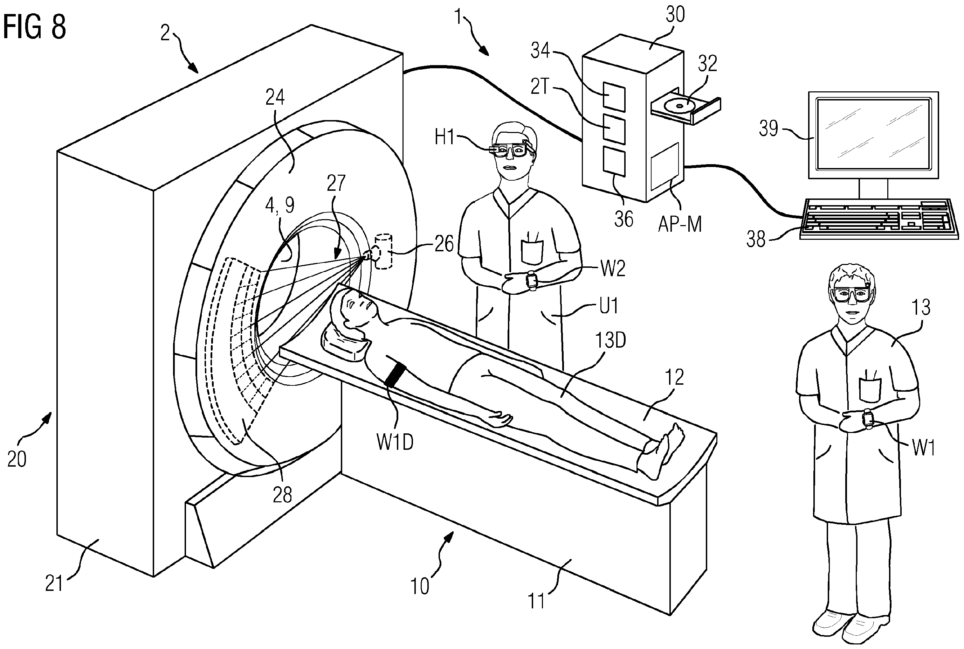

Fig. 8 zeigt ein System 1 mit einer medizinischen Bildgebungsvorrichtung 2 gemäß einer weiteren Ausführungsform der Erfindung. Ohne Beschränkung des allgemeinen Erfindungsgedankens ist für die medizinische Bildgebungsvorrichtung 2 beispielhaft ein Computertomographiegerät gezeigt. Die medizinische Bildgebungsvorrichtung 2 weist die Gantry 20, die tunnelförmige Öffnung 9, die Patientenlagerungsvorrichtung 10 und die Steuerungsvorrichtung 30 auf.

Die Gantry 20 weist den stationären Tragrahmen 21 und den Rotor 24 auf. Der Rotor 24 ist mittels einer Drehlagerungsvorrichtung an dem stationären Tragrahmen 21 um eine Rotationsachse relativ zu dem stationären Tragrahmen 21 drehbar angeordnet.

In die tunnelförmige Öffnung 9 ist ein Patient einführbar. In der tunnelförmigen Öffnung 9 befindet sich der Akquisitionsbereich 4. In dem Akquisitionsbereich 4 ist ein abzubildender Bereich eines Patienten derart positionierbar, dass die Strahlung 27 von der Strahlungsquelle 26 zu dem abzubildenden Bereich gelangen kann und nach einer Wechselwirkung mit dem abzubildenden Bereich zu dem Strahlungsdetektor 28 gelangen kann.

Die Patientenlagerungsvorrichtung 10 weist den Lagerungssockel 11 und die Lagerungsplatte 12 zur Lagerung eines Patienten auf. Die Lagerungsplatte 12 ist derart relativ zu dem Lagerungssockel 11 bewegbar an dem Lagerungssockel 11 angeordnet, dass die Lagerungsplatte 12 in einer Längsrichtung der Lagerungsplatte 12 in den Akquisitionsbereich 4 einführbar ist.

Die medizinische Bildgebungsvorrichtung 2 ist zur Akquisition von Akquisitionsdaten basierend auf einer elektromagnetischen Strahlung 27 ausgebildet. Die medizinische Bildgebungsvorrichtung 2 weist eine Akquisitionseinheit auf. Die Akquisitionseinheit ist eine Projektionsdaten-Akquisitionseinheit mit der Strahlungsquelle 26, z. B. einer Röntgenquelle, und dem Detektor 28, z. B. einem Röntgendetektor, insbesondere einem energieauflösenden Röntgendetektor. Die Strahlungsquelle 26 ist an dem Rotor 24 angeordnet und zur Emission einer Strahlung 27, z. B. einer Röntgenstrahlung, mit Strahlungsquanten 27 ausgebildet. Der Detektor 28 ist an dem Rotor 24 angeordnet und zur Detektion der Strahlungsquanten 27 ausgebildet. Die Strahlungsquanten 27 können von der Strahlungsquelle 26 zu dem abzubildenden Bereich eines Patienten gelangen und nach einer Wechselwirkung mit dem abzubildenden Bereich auf den Detektor 28 auftreffen. Auf diese Weise können mittels der Akquisitionseinheit Akquisitionsdaten des abzubildenden Bereichs in Form von Projektionsdaten erfasst werden.

Die Steuerungsvorrichtung 30 ist zum Empfangen der von der Akquisitionseinheit akquirierten Akquisitionsdaten ausgebildet. Die Steuerungsvorrichtung 30 ist zum Steuern der medizinischen Bildgebungsvorrichtung 2 ausgebildet. Die Steuerungsvorrichtung 30 ist ein Computer und weist das computerlesbare Medium 32 und das Prozessorsystem 36 auf.

Die Steuerungsvorrichtung 30 weist die Bildrekonstruktionseinrichtung 34 auf. Mittels der Bildrekonstruktionseinrichtung 34 kann basierend auf den Akquisitionsdaten ein medizinischer Bilddatensatz rekonstruiert werden.

Die medizinische Bildgebungsvorrichtung 2 weist eine Eingabevorrichtung 38 und eine Ausgabevorrichtung 39 auf, welche jeweils mit der Steuerungsvorrichtung 30 verbunden sind. Die Eingabevorrichtung 38 ist zum Eingeben von Steuerungs-Informationen, z. B. Bildrekonstruktionsparametern, Untersuchungsparametern oder ähnliches, ausgebildet. Die Ausgabevorrichtung 39 ist insbesondere zum Ausgeben von Steuerungs-Informationen, Bildern und/oder Tönen ausgebildet.

Die Steuerungsvorrichtung 30 der medizinischen Bildgebungsvorrichtung 2 weist die Untersuchungsparameter-Anpassungseinheit AP-M und das Datenübertragungsmodul 2T auf. Die Datenübertragungseinheit TD-M wird von dem Datenübertragungsmodul 2T und zumindest einem der Datenübertragungsmodule WT1, WT2 und 3T gebildet. Die Datenübertragungseinheit TD-M kann beispielsweise auf kabelloser Datenübertragung und/oder einem Funknetz basieren und/oder zumindest eine Basisstation, mit welcher die Datenübertragungsmodule WT1, WT2 und 3T jeweils verbunden sein können, aufweisen.

Die medizinische Information N, welche den Patienten 13 betrifft, und/oder eine medizinische Information, welche den Patienten 13D betrifft, kann beispielsweise mittels einer Datenbrille H1 in Form einer Erweiterte-Realität-Information in einem Sichtfeld der Bedienperson U1 ausgegeben werden. Der Patient 13D trägt an einem Oberarm das erste Wearable W1D in Form eines Fitnessarmbands.

## Patentansprüche

1. Verfahren zum Ausgeben einer medizinischen Information, welche eine Eignung eines Patienten für eine Untersuchung mittels einer medizinischen Bildgebungsvorrichtung (2) und/oder eine patientenspezifische Konfiguration einer medizinischen Bildgebungsvorrichtung (2) betrifft, wobei das Verfahren die folgenden Schritte umfasst:
- Erfassen (RD) einer Messdatenreihe, welche zumindest einen physiologischen Parameter des Patienten betrifft, mittels eines ersten Wearables (W1), welches von dem Patienten getragen wird,
- Ermitteln (DI) der medizinischen Information basierend auf der Messdatenreihe,
- Ausgeben (OI) der medizinischen Information.

2. Verfahren nach Anspruch 1,
- wobei das erste Wearable (W1) eine erste Datenverarbeitungseinheit aufweist,
- wobei eine erste Software-Applikation (A1) von der ersten Datenverarbeitungseinheit ausgeführt wird,
- wobei die medizinische Information mittels der ersten Software-Applikation (A1) ermittelt und/oder ausgegeben wird.

3. Verfahren nach einem der Ansprüche 1 bis 2, wobei das Verfahren ferner den folgenden Schritt umfasst:
- Bereitstellen (PR) einer Referenzinformation, welche eine Identifikation der Untersuchung und/oder der medizinischen Bildgebungsvorrichtung (2) betrifft und/oder welche eine untersuchungsspezifische und/oder gerätespezifische Bedingung an den zumindest einen physiologischen Parameter des Patienten betrifft,
- wobei die medizinische Information ferner basierend auf der Referenzinformation ermittelt wird.

4. Verfahren nach einem der Ansprüche 1 bis 3, wobei das Verfahren ferner den folgenden Schritt umfasst:
- Übertragen (TS) von Daten zwischen dem ersten Wearable (W1) und einem Datenverarbeitungssystem,
- wobei die Daten mindestens ein Element umfassen, welches aus der Gruppe gewählt ist, welche aus der Messdatenreihe, der medizinischen Information, der Referenzinformation und Kombinationen davon besteht,
- wobei das Datenverarbeitungssystem eine zweite Datenverarbeitungseinheit aufweist,
- wobei eine zweite Software-Applikation (A2) von der zweiten Datenverarbeitungseinheit ausgeführt wird,
- wobei die medizinische Information mittels der zweiten Software-Applikation (A2) ermittelt und/oder ausgegeben wird.

5. Verfahren nach einem der Ansprüche 1 bis 4,
- wobei das Datenverarbeitungssystem ein zweites Wearable (W2) aufweist, welches von einer Bedienperson getragen wird, und/oder
- wobei das zweite Wearable (W2) die zweite Datenverarbeitungseinheit aufweist.

6. Verfahren nach einem der Ansprüche 1 bis 5,
- wobei das erste Wearable (W1) eine erste Anzeigeeinheit aufweist und/oder wobei das Datenverarbeitungssystem eine zweite Anzeigeeinheit aufweist,
- wobei die medizinische Information mittels der ersten Anzeigeeinheit und/oder mittels der zweiten Anzeigeeinheit angezeigt wird.

7. Verfahren nach einem der Ansprüche 1 bis 6, wobei das Verfahren ferner den folgenden Schritt umfasst:
- Übertragen (TD) von Daten zwischen dem ersten Wearable (W1) und der medizinischen Bildgebungsvorrichtung (2) und/oder zwischen dem Datenverarbeitungssystem und der medizinischen Bildgebungsvorrichtung (2),
- wobei die Daten mindestens ein Element umfassen, welches aus der Gruppe gewählt ist, welche aus der Messdatenreihe, der medizinischen Information, der Referenzinformation und Kombinationen davon besteht.

8. Verfahren nach einem der Ansprüche 1 bis 7, wobei das Verfahren ferner den folgenden Schritt umfasst:
- Anpassen (AP) zumindest eines Untersuchungsparameters der medizinischen Bildgebungsvorrichtung (2) basierend auf der medizinischen Information.

9. Verfahren nach einem der Ansprüche 1 bis 8,
- wobei die medizinische Information eine erste Information umfasst, welche angibt, ob der Patient für die Untersuchung mittels der medizinischen Bildgebungsvorrichtung (2) geeignet ist, und/oder
- wobei die medizinische Information eine zweite Information umfasst, welche angibt, ob und/oder wie die Eignung des Patienten für die Untersuchung hergestellt und/oder verbessert werden kann, und/oder
- wobei die medizinische Information eine dritte Information umfasst, welche eine Kenngröße des physiologischen Parameters des Patienten und/oder ein Maß für eine Abweichung einer Kenngröße des physiologischen Parameters des Patienten von einer Bezugsgröße betrifft, und/oder
- wobei die medizinische Information eine vierte Information umfasst, welche angibt, ob und/oder wie die Konfiguration der medizinischen Bildgebungsvorrichtung (2) für die Untersuchung des Patienten patientenspezifisch optimiert werden kann.

10. Verfahren nach einem der Ansprüche 1 bis 9,
- wobei die Messdatenreihe eine Herzaktivität des Patienten betrifft.

11. System (1), aufweisend
- ein erstes Wearable (W1) mit einer Erfassungseinheit (RD-M), welche zum Erfassen (RD) einer Messdatenreihe, welche zumindest einen physiologischen Parameter des Patienten betrifft, ausgebildet ist,
- eine Ermittlungseinheit (DI-M), welche zum Ermitteln (DI) einer medizinischen Information basierend auf der Messdatenreihe ausgebildet ist, wobei die medizinische Information eine Eignung eines Patienten für eine Untersuchung mittels einer medizinischen Bildgebungsvorrichtung (2) und/oder eine patientenspezifische Konfiguration einer medizinischen Bildgebungsvorrichtung (2) für eine Untersuchung eines Patienten betrifft,
- eine Ausgabeeinheit (OI-M), welche zum Ausgeben (OI) der medizinischen Information ausgebildet ist.

12. System (1) nach Anspruch 11, ferner aufweisend
- ein Datenverarbeitungssystem,
- eine Datenübertragungseinheit (TS-M), welche zum Übertragen (TS) von Daten zwischen dem ersten Wearable (W1) und dem Datenverarbeitungssystem ausgebildet ist,
- wobei das Datenverarbeitungssystem die Ermittlungseinheit (DI-M) und/oder die Ausgabeeinheit (OI-M) aufweist.

13. System (1) nach einem der Ansprüche 11 bis 12,
- wobei das erste Wearable (W1) die Ermittlungseinheit (DI-M) und/oder die Ausgabeeinheit (OI-M) aufweist und/oder
- wobei das Datenverarbeitungssystem ein zweites Wearable (W2) aufweist, welches die Ermittlungseinheit (DI-M) und/oder die Ausgabeeinheit (OI-M) aufweist.

14. System (1) nach einem der Ansprüche 11 bis 13, ferner aufweisend
- die medizinische Bildgebungsvorrichtung (2),
- eine Datenübertragungseinheit (TD-M), welche zum Übertragen (TD) von Daten zwischen dem ersten Wearable (W1) und der medizinischen Bildgebungsvorrichtung (2) und/oder zwischen dem Datenverarbeitungssystem und der medizinischen Bildgebungsvorrichtung (2) ausgebildet ist,
- eine Untersuchungsparameter-Anpassungseinheit (AP-M), welche zum Anpassen (AP) zumindest eines Untersuchungsparameters der medizinischen Bildgebungsvorrichtung (2) basierend auf der medizinischen Information ausgebildet ist.

15. Verwendung eines ersten Wearables (W1) zum Ausgeben einer medizinischen Information, welche eine Eignung eines Patienten für eine Untersuchung mittels einer medizinischen Bildgebungsvorrichtung (2) und/oder eine patientenspezifische Konfiguration einer medizinischen Bildgebungsvorrichtung (2) für eine Untersuchung eines Patienten betrifft,
- wobei eine Messdatenreihe, welche zumindest einen physiologischen Parameter des Patienten betrifft, mittels des ersten Wearables (W1), welches von dem Patienten getragen wird, erfasst wird,
- wobei die medizinische Information basierend auf der Messdatenreihe ermittelt wird,
- wobei die medizinische Information ausgegeben wird.

16. Verwendung eines Systems (1), aufweisend ein erstes Wearable (W1) und ein zweites Wearable (W2), zum Ausgeben einer medizinischen Information, welche eine Eignung eines Patienten für eine Untersuchung mittels einer medizinischen Bildgebungsvorrichtung (2) und/oder eine patientenspezifische Konfiguration einer medizinischen Bildgebungsvorrichtung (2) für eine Untersuchung eines Patienten betrifft,
- wobei eine Messdatenreihe, welche zumindest einen physiologischen Parameter des Patienten betrifft, mittels des ersten Wearables (W1), welches von dem Patienten getragen wird, erfasst wird,
- wobei die medizinische Information basierend auf der Messdatenreihe ermittelt wird,
- wobei die medizinische Information mittels des zweiten Wearables (W2) ausgegeben wird.
